# EUROPÄISCHE PATENTANMELDUNG

(11) **EP 2 860 524 A1**
(43) Veröffentlichungstag der Anmeldung: **15.04.2015**
(21) Anmeldenummer: 14188084.9
(22) Anmeldetag: 08.10.2014
(51) Int. Cl.: G01N 33/00

(54) **Messsystem mit Ammoniaksensor zum Einsatz in Tierställen**

(30) Priorität: 08.10.2013 DE 102013016595
(71) Anmelder: ExTox Gasmess-Systeme GmbH, 59423 Unna (DE)
(72) Erfinder: Unruh, Michael, 45731 Waltrop (DE)
(74) Vertreter: Isfort, Olaf

(57) **Zusammenfassung**

Die Erfindung betrifft ein Messsystem zum Messen des Ammoniakgehalts in der Umgebungsluft (3), insbesondere in Tierställen, mit einem elektrochemischen Sensor (5) zum Messen des Ammoniakgehalts der Umgebungsluft (3), und mit einer Pumpe (2), die die Umgebungsluft (3) dem Sensor (5) zuführt. Es ist Aufgabe der Erfindung, ein Messsystem bereitzustellen, das geeignet ist, über einen längeren Zeitraum in einer ammoniakhaltigen Atmosphäre den Ammoniakgehalt in der Luft zu messen. Diese Aufgabe löst die Erfindung dadurch, dass eine Filtereinheit (6) vorgesehen ist, die ein Absorptionsmittel zum Absorbieren des Ammoniaks aus der Umgebungsluft (3) und ein Trägermaterial zum Binden des Absorptionsmittels enthält, wobei es sich bei dem Absorptionsmittel um eine polare Flüssigkeit handelt. Eine schaltbare Ventilanordnung (4) ist vorgesehen, wobei in einer Schaltposition die Umgebungsluft (3) dem Sensor (5) direkt zugeführt wird und in einer anderen Schaltposition die Umgebungsluft (3) zunächst die Filtereinheit(6) durchströmt und danach dem Sensor (5) zugeführt wird.

## Beschreibung

Die Erfindung betrifft ein Messsystem zum Messen des Ammoniakgehalts in der Umgebungsluft, insbesondere in Tierställen, mit einem elektrochemischen Sensor zum Messen des Ammoniakgehalts der Umgebungsluft, und mit einer Pumpe, die die Umgebungsluft dem Sensor zuführt.

Elektrochemische Sensoren zum Messen des Ammoniakgehalts in der Umgebungsluft werden in Tierställen, insbesondere in Geflügelställen, eingesetzt, wo Ammoniak entsteht. Dort darf die Ammoniakkonzentration in der Luft einen kritischen Wert nicht überschreiten. Außerdem muss die Abgabe von Ammoniak an die Umwelt überwacht werden. In Ställen mit starker Ammoniakbelastung muss die Abluft durch geeignete Wäscher behandelt werden, bevor sie in die Umwelt abgegeben wird. Dies setzt eine zuverlässige und gut funktionierende Messung des Ammoniakgehalts in der Luft voraus.

Bekannte Ammoniaksensoren arbeiten nach einem elektrochemischen Prinzip. Die kommerziell verfügbaren elektrochemischen Zellen haben den Vorteil, dass sie sehr selektiv und empfindlich auf Ammoniak ansprechen. Nachteilig ist, dass die elektrochemischen Zellen nicht dauerhaft einer ammoniakhaltigen Atmosphäre ausgesetzt sein dürfen. Bei dauerhafter Beaufschlagung mit Ammoniak haben die elektrochemischen Zellen sehr schnell ihre maximale Lebensdauer erreicht. Ein elektrochemischer Ammoniaksensor, der beispielsweise eine Standzeit von ca. 15000 ppm*h hat und ständig einem Ammoniakpegel von nur 20 ppm ausgesetzt ist, erreicht eine Lebensdauer von lediglich 750 h; das entspricht etwa einem Monat. Für den Einsatz in Tierställen muss die Lebensdauer aber wenigstens 6 Monate betragen.

Der Erfindung liegt daher die Aufgabe zugrunde, ein Messsystem bereitzustellen, das geeignet ist, über einen längeren Zeitraum in einer ammoniakhaltigen Atmosphäre den Ammoniakgehalt in der Luft zu messen.

Die Aufgabe wird ausgehend von einem Messsystem der eingangs genannten Art dadurch gelöst, dass eine Filtereinheit vorgesehen ist, die ein Absorptionsmittel zum Absorbieren des Ammoniaks aus der Umgebungsluft und ein Trägermaterial zum Binden des Absorptionsmittels enthält, wobei es sich bei dem Absorptionsmittel um eine polare Flüssigkeit handelt. Das erfindungsgemäße Messsystem weist des Weiteren eine schaltbare Ventilanordnung auf, wobei in einer Schaltposition die Umgebungsluft dem Sensor direkt zugeführt wird und in einer anderen Schaltposition die Umgebungsluft zunächst die Filtereinheit durchströmt und danach dem Sensor zugeführt wird.

Die Erfindung basiert auf der Erkenntnis, dass bei der Ammoniaküberwachung in Tierställen die Messung nicht permanent erfolgen muss. Es ist z. B. ausreichend, einmal in der Stunde oder auch nur einmal in zwei Stunden eine Messung vorzunehmen. Vor diesem Hintergrund ist das erfindungsgemäße Messsystem in der Lage, den elektrochemischen Ammoniaksensor in einer Schaltposition der Ventilanordnung nur für kurze Zeit mit der Stallluft zu beaufschlagen, um den Ammoniakgehalt zu messen. Unmittelbar nach dem Messvorgang wird dem elektrochemischen Sensor in der anderen Schaltposition eine ammoniakfreie, zumindest ammoniakarme Luft zugeführt. Dies geschieht dadurch, dass in der anderen Schaltposition die Umgebungsluft zunächst die Filtereinheit durchströmt und erst danach dem Sensor zugeführt wird. In der Filtereinheit wird die Umgebungsluft von Ammoniak befreit, zumindest wird der Ammoniakgehalt stark reduziert, bevor die so behandelte Umgebungsluft den elektrochemischen Sensor erreicht. Der Sensor befindet sich auf diese Weise während der meisten Zeit unter ammoniakfreier oder -armer Atmosphäre. Die Lebensdauer des Sensors erhöht sich dadurch erheblich.

Ein weiterer Vorteil des erfindungsgemäßen Messsystems ist, dass es außer der Umgebungsluft keine weiteren Fluide benötigt, um eine zuverlässige Messfunktion über einen längeren Zeitraum zu gewährleisten.

Als Absorptionsmittel kommt gemäß der Erfindung eine polare Flüssigkeit, insbesondere Wasser, zum Einsatz. Denn eine besondere Eigenschaft des Ammoniaks ist die hohe Löslichkeit in polaren Flüssigkeiten wie Wasser. Ammoniak wird in der Filtereinheit durch die darin befindliche polare Flüssigkeit der Umgebungsluft entzogen. Auf diese Weise absorbiert das Absorptionsmittel den Ammoniak im Sinne der Erfindung.

Um die polare Flüssigkeit zu binden, kommt gemäß der Erfindung ein Trägermaterial zum Einsatz. Das Trägermaterial sorgt dafür, dass die Flüssigkeit nicht auslaufen kann. Gleichzeitig stellt das Trägermaterial eine große Oberfläche zur Verfügung, über die die Umgebungsluft mit der gebundenen polaren Flüssigkeit in Kontakt treten kann, um so die Ammoniakabsorption hinreichend effektiv zu bewirken. Durch das Trägermaterial ist außerdem eine praktische Handhabe der polaren Flüssigkeit gewährleistet.

Ein besonders geeignetes Trägermaterial ist Silikagel, da es die Eigenschaft besitzt, große Mengen Wasser zu binden. Das pulver- oder granulatförmige Silikagel weist außerdem eine große Oberfläche auf, was die möglichst vollständige Absorption des Ammoniak in der Filtereinheit begünstigt. Durch die Verwendung von Silikagel wird eine Kapazität für die von der Filtereinheit absorbierbare Ammoniakmenge erreicht, die sehr lange Standzeiten der Filtereinheit und des elektrochemischen Ammoniaksensors ermöglicht.

Außerdem weist das Silikagel bei eintretender Trocknung durch beigefügte Indikatoren einen Farbwechsel auf. Die Filterfunktion kann somit auf einfachste Weise kontrolliert und ggf. durch erneutes Befeuchten wiederhergestellt werden.

Die schaltbare Ventilanordnung weist vorzugsweise ein Magnetventil auf. In einer besonders bevorzugten Ausführungsform handelt es sich um ein 3/2-Wegeventil, das drei Anschlüsse - zwei Eingänge, ein Ausgang - und zwei Ventilsitze aufweist. Durch den ersten Eingang kann somit die ungefilterte (Messgas) und durch den zweiten Eingang die gefilterte Umgebungsluft (Spülgas) einströmen. Es ist lediglich ein Ausgang nötig, da je nach Bedarf entweder das Messgas oder das Spülgas dem Sensor zugeführt wird.

Bevorzugt werden das Trägermaterial und das Absorptionsmittel in eine Filterkartusche eingebracht. Die Verwendung einer Filterkartusche hat den Vorteil, dass die Umgebungsluft zusätzlich von Verschmutzungen, Staubanfall, Kondensat, etc. befreit wird.

In einer Ausführungsform des erfindungsgemäßen Messsystems befindet sich der Sensor zumindest teilweise in einem Transmittergehäuse. Da die Umgebungsluft in das Transmittergehäuse eindringen kann, kann es sinnvoll sein, dass sich wenigstens eine zweite Filtereinheit im Transmittergehäuse befindet, welche den Sensor zumindest teilweise umhüllt. Dies kann dadurch realisiert werden, dass Hohlräume in dem Transmittergehäuse in der Umgebung des Sensors, insbesondere an dessen Rückseite, mit Trägermaterial (Silikagel) und Absorptionsmittel (Wasser) gefüllt werden.

Bevorzugt ist ein erfindungsgemäßes Messsystem, bei dem die Pumpe die Umgebungsluft nach der Messung des Ammoniakgehalts in die Umgebung zurückführt, sodass die Umgebungsluft nicht in einem geschlossenen Kreislauf zirkuliert.

Außerdem betrifft die Erfindung ein Verfahren zum Messen des Ammoniakgehalts in der Umgebungsluft, insbesondere in Tierställen, unter Verwendung eines erfindungsgemäßen Messsystems. Dabei wird die Ventilanordnung für einen Messvorgang in diejenige Schaltposition geschaltet, in der die Umgebungsluft direkt dem elektrochemischen Sensor zugeführt wird. In dieser Schaltposition wird der Ammoniakgehalt der Umgebungsluft gemessen. Die Schaltposition wird während eines begrenzten Zeitintervalls beibehalten, und zwar wenigstens so lange bis ein stabiler Messwert vorliegt. Danach wird auf diejenige Schaltposition umgeschaltet, in der die Umgebungsluft zunächst die Filtereinheit durchströmt und danach dem elektrochemischen Sensor zugeführt wird. Dieser Vorgang kann nach einem vorgegebenen Zeitschema wiederholt werden, um den Ammoniakgehalt in der Umgebungsluft zu überwachen.

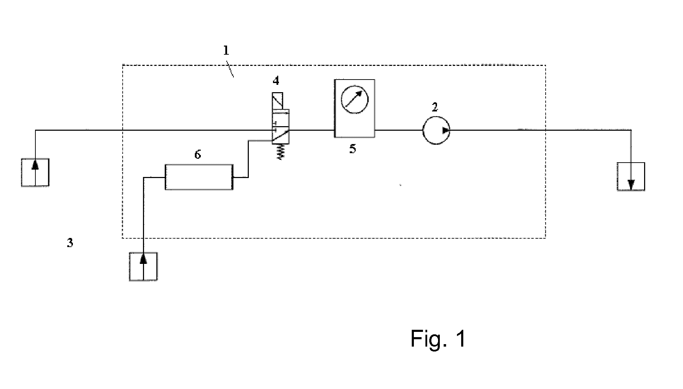

Ein Ausführungsbeispiel der Erfindung wird im Folgenden anhand der Zeichnung näher erläutert.

Die Zeichnung zeigt einen schematischen Gasplan eines Messsystems 1 gemäß der Erfindung. Durch eine Pumpe 2 wird Umgebungsluft 3 in das Messsystem 1 eingesogen. Je nach Schaltposition eines Magnetventils 4 wird die Umgebungsluft 3 direkt einem elektrochemischen Ammoniaksensor 5 zugeführt, oder die Umgebungsluft 3 durchströmt zunächst eine Filtereinheit 6 und wird danach dem elektrochemischen Ammoniaksensor 5 zugeführt. Nachdem die Umgebungsluft 3 den elektrochemischen Ammoniaksensor 5 passiert hat, wird diese mittels der Pumpe 2 aus dem Messsystem 1 wieder in die Umgebung abgeführt. Die Filtereinheit 6 umfasst eine Filterkartusche (nicht dargestellt), in der sich als Absorptionsmittel zum Absorbieren des Ammoniaks aus der Umgebungsluft Wasser befindet, das in Silikagel als Trägermaterial gebunden ist.

Das Magnetventil 4 wird jeweils kurzzeitig in diejenige Schaltposition geschaltet, in der die Umgebungsluft 3 direkt dem Ammoniaksensor 5 zugeführt wird. In dieser Schaltposition wird der Ammoniakgehalt der Umgebungsluft 3 gemessen. Die Schaltposition wird jeweils nur solange beibehalten, bis ein zuverlässiger und stabiler Messwert vorliegt. Danach wird auf diejenige Schaltposition umgeschaltet, in der die Umgebungsluft 3 zunächst die Filtereinheit 6 durchströmt und danach dem Sensor 5 zugeführt wird. Dies stellt sicher, dass der Ammoniaksensor 5 während eines größeren Teils der Gesamtzeit mit ammoniakfreier oder zumindest -armer Umgebungsluft 3 beaufschlagt wird. Dieser Zyklus wird wiederholt, so dass z.B. einmal pro Stunde oder einmal in zwei Stunden der Ammoniakgehalt der Umgebungsluft ermittelt wird.

## Patentansprüche

1. Messsystem zum Messen des Ammoniakgehalts in der Umgebungsluft (3), insbesondere in Tierställen, mit einem elektrochemischen Sensor (5) zum Messen des Ammoniakgehalts der Umgebungsluft (3), und mit einer Pumpe (2), die die Umgebungsluft (3) dem Sensor (5) zuführt,
**gekennzeichnet durch**
- eine Filtereinheit (6), die ein Absorptionsmittel zum Absorbieren des Ammoniaks aus der Umgebungsluft und ein Trägermaterial zum Binden des Absorptionsmittels enthält, wobei es sich bei dem Absorptionsmittel um eine polare Flüssigkeit handelt, und
- eine schaltbare Ventilanordnung (4), wobei in einer Schaltposition die Umgebungsluft (3) dem Sensor (5) direkt zugeführt wird und in einer anderen Schaltposition die Umgebungsluft (3) zunächst die Filtereinheit (6) durchströmt und danach dem Sensor (5) zugeführt wird.

2. Messsystem nach Anspruch 1, **dadurch gekennzeichnet, dass** die polare Flüssigkeit Wasser ist.

3. Messsystem nach Anspruch 1 oder 2, **dadurch gekennzeichnet, dass** das Trägermaterial Silikagel ist.

4. Messsystem nach einem der vorherigen Ansprüche, **dadurch gekennzeichnet, dass** die schaltbare Ventilanordnung (4) ein Magnetventil aufweist, vorzugsweise ein 3/2-Wegeventil.

5. Messsystem nach einem der vorherigen Ansprüche, **dadurch gekennzeichnet, dass** sich das Trägermaterial und das Absorptionsmittel in einer Filterkartusche befinden.

6. Messsystem nach einem der vorherigen Ansprüche, **dadurch gekennzeichnet, dass** sich der elektrochemische Sensor (5) in einem Transmittergehäuse befindet.

7. Messsystem nach Anspruch 6 **dadurch gekennzeichnet, dass** sich in dem Transmittergehäuse eine zweite Filtereinheit befindet, die den elektrochemischen Sensors (5) zumindest teilweise umhüllt.

8. Messsystem nach einem der vorherigen Ansprüche, **dadurch gekennzeichnet, dass** die Pumpe (2) die Umgebungsluft (3) nach der Messung des Ammoniakgehalts in die Umgebung zurückführt, sodass die Umgebungsluft (3) nicht in einem geschlossenen Kreislauf zirkuliert.

9. Verfahren zum Messen des Ammoniakgehalts in der Umgebungsluft (3), insbesondere in Tierställen, unter Verwendung eines Messsystems nach einem der Ansprüche 1 bis 8, **dadurch gekennzeichnet, dass**
- mittels der Pumpe (2) Umgebungsluft (3) in das Messsystem (1) eingesogen wird,
- je nach Schaltposition der Ventilanordnung (4) die Umgebungsluft (3) direkt dem elektrochemischen Sensor (5) zugeführt wird, oder die Umgebungsluft (3) zunächst die Filtereinheit (6) durchströmt und danach dem elektrochemischen Sensor (5) zugeführt wird,
- die Umgebungsluft (3) mittels der Pumpe (2) aus dem Messsystem (1) wieder in die Umgebung abgeführt wird, nachdem sie den elektrochemischen Sensor (5) passiert hat,
wobei die Ventilanordnung (4) für einen Messvorgang in diejenige Schaltposition geschaltet wird, in der die Umgebungsluft (3) direkt dem elektrochemischen Sensor (5) zugeführt wird, und in dieser Schaltposition der Ammoniakgehalt der Umgebungsluft (3) gemessen wird, wobei diese Schaltposition während eines Zeitintervalls beibehalten wird und danach auf diejenige Schaltposition umgeschaltet wird, in der die Umgebungsluft (3) zunächst die Filtereinheit (6) durchströmt und danach dem elektrochemischen Sensor (5) zugeführt wird.
